# EUROPEAN PATENT APPLICATION

(11) **EP 1 930 028 A1**
(43) Date of publication of application: **11.06.2008**
(21) Application number: 06781899.7
(22) Date of filing: 28.07.2006
(51) Int. Cl.: A61K 47/32, A61K 9/14, A61K 9/18, A61K 9/24, A61K 31/573, A61K 47/38, A61P 27/02

(54) **NON-INVASIVE DRUG DELIVERY SYSTEM TARGETING POSTERIOR EYE TISSUE USING SOLID COMPOSITION**

(30) Priority: 29.07.2005 JP 2005221388
(71) Applicant: SANTEN PHARMACEUTICAL CO., LTD., Higashiyodogawa-ku, Osaka-shi, Osaka 533-8651 (JP)
(72) Inventor: OKABE, Koumei c/o SANTEN PHARMACEUTICAL CO., LTD., Ikoma-shi, Nara 630-0101 (JP); TASAKA, Fumitaka c/o SANTEN PHARMACEUTICAL CO., LTD., Osaka-shi, Osaka 533-8651 (JP)
(74) Representative: Müller-Boré & Partner Patentanwälte
(86) International application number: PCT/JP2006/314989
(87) International publication number: WO 2007/013590

(57) **Abstract**

The present invention provides a drug delivery system by noninvasive administration, which is excellent in drug transfer to a tissue of the posterior segment of the eye via a local eye tissue. By administering a solid composition comprising a drug and a mucoadhesive substance having an adhesion strength of from 200 to 1000 g in the conjunctival sac, a drug delivery system excellent in drug transfer to a tissue of the posterior segment of the eye via a local eye tissue can be constructed.

## Description

### Technical Field

The present invention relates to a noninvasive drug delivery system to a tissue of the posterior segment of the eye such as a retina, a choroid, a sclera, an optic nerve, a tissue around the optic nerve or a vitreous body.

### Background Art

Many diseases of tissues of the posterior segment of the eye such as a retina, a choroid, a sclera, an optic nerve, a tissue around the optic nerve and a vitreous body are intractable diseases, and not a few diseases among them show serious symptoms which may cause visual loss. Typical examples of such a disease include age-related macular degeneration, diabetic retinopathy, diabetic macular edema, uveitis, retinitis pigmentosa, proliferative vitreoretinopathy, central retinal vein occlusion, branch retinal vein occlusion, central retinal artery occlusion, branch retinal artery occlusion, retinal detachment, cytomegalovirus retinitis, optic nerve disorders accompanying glaucoma and the like. All these diseases may cause reduced vision or visual loss, and development of an effective drug treatment method for such diseases of tissues of the posterior segment of the eye has been demanded.

Examples of an effective drug for diseases of the posterior segment of the eye include many drugs, for example, steroids such as betamethasone and dexamethasone, antiinflammatory agents such as bromofenac, antibacterial agents such as quinolone compounds and erythromycin, antiviral agents such as ganciclovir and acyclovir, anticancer agents such as methotrexate and MMP inhibitors, angiogenesis inhibitors such as endostatin and VEGF inhibitors, neuroprotective agents such as MK-801 and timolol, antioxidants such as catechin and vitamin E, bone resorption inhibitors such as bisphosphonate, agents involved in the maintenance of visual function such as retinoic acid and its derivatives, and the like. Many useful drugs have been approved, however, a tissue of the posterior segment of the eye, which is a diseased area, is located in the back of the eye, and it is very difficult to transfer a drug to the area, which was a problem in the development of drug treatment method for diseases of the posterior segment of the eye.

As for the drug treatment of eye diseases, a treatment by instillation administration is most commonly performed and is highly convenient. However, the instillation administration has a problem that a drug is difficult to transfer to a tissue of the posterior segment of the eye.

As the administration method of a drug for diseases of the posterior segment of the eye, systemic administration such as intravenous injection or oral administration has been attempted. However, by such an administration method, the amount of a drug transferred to a tissue of the posterior segment of the eye, which is a diseased area, is small. Therefore, it is necessary to administer the drug at high dose and high frequency in order to deliver a sufficient amount of the drug to a tissue of the posterior segment of the eye, and it has a problem in terms of efficiency from the viewpoint of drug treatment of a tissue of the posterior segment of the eye.

On the other hand, administration methods such as intravitreal, sub-Tenon or subconjunctival injection and implant have also been attempted for the drug treatment method for diseases of the posterior segment of the eye. For example, Journal of ocular pharmacology and therapeutics, (2001) 17/4 393-401 describes a drug delivery system to a tissue of the posterior segment of the eye by intravitreal injection. JP-A-2003-313119 and JP-A-2005-097275 describe a drug delivery system to a tissue of the posterior segment of the eye by subconjunctival administration of controlled-release microparticles containing a drug and a drug delivery system to a tissue of the posterior segment of the eye by sub-Tenon administration of microparticles containing a drug. Further, JP-T-2004-535886 describes a biocompatible implant for transferring a drug to a tissue of the posterior segment of the eye. In these administration methods, a drug is directly administered to or implanted in a diseased area or a tissue close to the diseased area, therefore, these methods have an advantage that a sufficient amount of a drug can be transferred to the diseased area. At the same time, because these are an administration method and a delivery technique involved in invasion in eye tissues, a patient sometimes feels stress or pain upon administration. Further, these administration methods cannot be performed by a patient per se, and are required to be performed by a doctor exclusively, therefore, they have a problem in terms of convenience.

### Disclosure of the Invention

### Problems to be Solved

Accordingly, development of a noninvasive drug delivery system which does not show tissue invasiveness upon administration accompanying an administration method such as injection or implant and can efficiently transfer a drug to a diseased area has been demanded.

That is, it is an important subject to develop a drug delivery system by noninvasive administration, which is excellent in drug transfer to a tissue of the posterior segment of the eye.

### Means for Solving the Problems

The present inventors made intensive studies and as a result, they surprisingly found that a means of administering a solid composition comprising a drug and a mucoadhesive substance having an adhesion strength of from 200 to 1000 g in the conjunctival sac is very useful as a noninvasive drug delivery system excellent in drug transfer to a tissue of the posterior segment of the eye.

Because the drug delivery system of the present invention is a noninvasive drug delivery system, it does not show tissue invasiveness by administration unlike an administration method commonly used in the medical field such as injection or implant. Further, a patient does not feel stress or pain accompanying the administration through injection or implant.

The solid composition to be used in the drug delivery system of the present invention is to be administered in the conjunctival sac, and does not require a professional technique upon administration unlike injection or implant which is commonly used in the medical field conventionally, therefore, it is excellent in terms of convenience.

That is, the present invention relates to:
(1) a noninvasive drug delivery system to a tissue of the posterior segment of the eye, characterized by administering a solid composition containing a drug and a mucoadhesive substance in the conjunctival sac, wherein the adhesion strength of the mucoadhesive substance is in the range of from 200 to 1000 g;
(2) a solid composition, which contains a drug and a mucoadhesive substance and is to be administered in the conjunctival sac, wherein transfer of the drug to a tissue of the posterior segment of the eye is improved due to the incorporation of the mucoadhesive substance and the adhesion strength of the mucoadhesive substance of from 200 to 1000 g;
(3) the drug delivery system according to the above (1) or the solid composition according to the above (2), wherein the adhesion strength of the mucoadhesive substance is in the range of from 300 to 900 g;
(4) the noninvasive drug delivery system or solid composition according to the above (3), wherein the transfer of the drug to a tissue of the posterior segment of the eye is transfer via a local eye tissue;
(5) the noninvasive drug delivery system or solid composition according to the above (3), wherein the transfer via a local eye tissue is transfer via a conjunctival tissue and a scleral tissue;
(6) the noninvasive drug delivery system or solid composition according to the above (3), wherein the mucoadhesive substance is one member or a combination of a plurality of members selected from polyacrylic acid derivatives, cellulose derivatives and salts thereof;
(7) the noninvasive drug delivery system or solid composition according to the above (6), wherein the polyacrylic acid derivative is a carboxyvinyl polymer or polyacrylic acid;
(8) the noninvasive drug delivery system or solid composition according to the above (6), wherein the cellulose derivative is hydroxypropyl cellulose;
(9) the noninvasive drug delivery system or solid composition according to the above (3), wherein the mucoadhesive substance is one member or a combination of a plurality of members selected from carboxyvinyl polymers and hydroxypropyl cellulose;
(10) the noninvasive drug delivery system or solid composition according to the above (3), wherein the tissue of the posterior segment of the eye is a retina, a choroid, a sclera, an optic nerve, a tissue around the optic nerve or a vitreous body;
(11) the noninvasive drug delivery system or solid composition according to the above (3), wherein the drug is a drug for treating or preventing a disease of a retina, a choroid, a sclera, an optic nerve, a tissue around the optic nerve or a vitreous body;
(12) the noninvasive drug delivery system or solid composition according to the above (3), wherein the drug is an antiinflammatory agent, an immunosuppressive agent, an antiviral agent, an anticancer agent, an antithrombotic agent, an angiogenesis inhibitor, an optic neuroprotective agent, an antibacterial agent, an antifungal agent or an antioxidant; and
(13) the noninvasive drug delivery system or solid composition according to the above (3), wherein the solid composition is in the form of a film, a pellet, a tablet, a rod, a particle or a sphere.

The mucoadhesive substance to be used in the present invention means a substance having a property that it is softened, swollen or the like by coming into contact with the lacrimal fluid after being administered in the conjunctival sac and adheres to a conjunctival tissue. Specific examples thereof include polyacrylic acid derivatives and salts thereof such as carboxyvinyl polymers, polyacrylic acid and sodium polyacrylate; polyvinyl alcohol, polyvinylpyrrolidone; cellulose derivatives and salts thereof such as hydroxyalkyl cellulose derivatives (including hydroxyethyl cellulose, hydroxypropyl cellulose and the like), hydroxyalkyl alkyl cellulose derivatives (including hydroxyethyl methyl cellulose, hydroxypropyl methyl cellulose and the like), alkyl cellulose derivatives (including methyl cellulose, ethyl cellulose and the like), carboxyalkyl cellulose derivatives and salts thereof (including carboxymethyl cellulose, sodium carboxymethyl cellulose and the like); alginic acid and salts thereof; polysaccharides such as chitosan, agar, dextran, gellan gum, xanthan gum and pectin, and derivatives thereof, combinations thereof and the like.

The adhesion strength of the mucoadhesive substance of the present invention means, although it will be described in detail in Test 5 mentioned below, a value obtained by the following measurement method. (1) Prepare a disc of a mucoadhesive substance and two silicone plugs (φ 14 mm). (2) Place 100 µL of physiological saline dropwise on one of the silicone plugs, and place the disc of a mucoadhesive substance thereon. Then, further place 100 µL of physiological saline dropwise on the disc, and place the remaining silicone plug thereon. Then, allow these to stand still for 1 minute as such, and then, compress them with a force of 667 g for a predetermined time (10 minutes) thereby to adhere to one another. (3) Measure the force when the adhesion is released using a force gauge (manufactured by Imada Inc.). The obtained value is determined to be the adhesion strength (g) of the mucoadhesive substance. The adhesion strength of the mucoadhesive substance is preferably in the range of from 200 to 1000 g, more preferably from 250 to 900 g, further more preferably from 300 to 900 g. When it is less than 200 g, the adhesion strength is low, and therefore a desired drug transfer cannot be obtained. When it exceeds 1000 g, it is not preferred from a production aspect.

The mucoadhesive substance having an adhesion strength of from 200 to 1000 g of the present invention means a mucoadhesive substance whose adhesion strength is in the range of from 200 to 1000 g. Specific examples thereof include carboxyvinyl polymers, polyvinyl alcohol, hydroxyethyl cellulose, hydroxypropyl cellulose, hydroxypropyl methyl cellulose, carboxymethyl cellulose, and salts thereof, polyacrylic acid and salts thereof, combinations thereof and the like, and preferred are carboxyvinyl polymers, polyvinyl alcohol, hydroxyethyl cellulose, hydroxypropyl cellulose, hydroxypropyl methyl cellulose, sodium carboxymethyl cellulose, polyacrylic acid, and combinations thereof, and more preferred are carboxyvinyl polymers, hydroxypropyl cellulose, polyacrylic acid, and a combination of a carboxyvinyl polymer and hydroxypropyl cellulose (at a weight ratio of from 100:1 to 1:100, preferably from 10:1 to 1:10, more preferably from 3:1 to 1:3).

In the present invention, the blending amount of the mucoadhesive substance varies depending on the type of the substance to be used in the solid composition, and is appropriately selected according to the type of the drug to be incorporated, the effective therapeutic concentration of drug or the like.

The drug delivery system of the present invention is excellent in drug transfer to a tissue of the posterior segment of the eye, and is particularly excellent in direct drug transfer to a tissue of the posterior segment of the eye. Here, the direct drug transfer to a tissue of the posterior segment of the eye means that a drug transfers to a tissue of the posterior segment of the eye not via a systemic route, but via a local eye tissue. That is, the drug delivery system of the present invention is excellent in drug transfer to a tissue of the posterior segment of the eye via a local eye tissue. Particularly, the drug delivery system of the present invention is excellent in drug transfer to a tissue of the posterior segment of the eye via a conjunctival tissue and a scleral tissue.

The solid composition according to the present invention is in a solid state when it is administered in the conjunctival sac, however, after administration, it is softened, swollen or the like by coming into contact with the lacrimal fluid and adheres to a conjunctival tissue. Once the composition adheres to a conjunctival tissue, it is not easily removed from the adhered area by blinking or the like. It is considered that by the adhesion of the solid composition of the present invention to a conjunctival tissue in this way, a state in which the lacrimal fluid present between the composition and conjunctival tissue is seldom replaced can be maintained, and further a state in which a drug is present on the conjunctival tissue at a high concentration can be maintained. It is presumed that this improves direct drug transfer to a tissue of the posterior segment of the eye via a local eye tissue by utilizing a concentration gradient of drug. In contrast, it is presumed that a solid composition which does not have mucoadhesiveness can retain the composition in the conjunctival sac for a predetermined time by being administered in the conjunctival sac, however, because a state as described above cannot be maintained on a conjunctival tissue, direct drug transfer to a tissue of the posterior segment of the eye via a local eye tissue is not improved. Further, it is presumed that even in the case of a solid composition with mucoadhesiveness, if its adhesion strength is low, a state as described above cannot be maintained on a conjunctival tissue, therefore, direct drug transfer to a tissue of the posterior segment of the eye via a local eye tissue is not improved.

The drug delivery system of the present invention is used for treating or preventing a disease of a tissue of the posterior segment of the eye, particularly a retina, a choroid, a sclera, an optic nerve, a tissue around the optic nerve or a vitreous body. Specific examples of the disease include inflammations due to various causes, viral or bacterial infections, diseases caused by choroidal and retinal neovascularization, diseases caused by retinal ischemia and optic nerve disorders caused by glaucoma. More specific examples thereof include uveitis, cytomegalovirus retinitis, age-related macular degeneration, diabetic retinopathy, diabetic macular edema, proliferative vitreoretinopathy, retinal detachment, retinitis pigmentosa, central retinal vein occlusion, branch retinal vein occlusion, central retinal artery occlusion, branch retinal artery occlusion and the like.

The drug to be incorporated in the solid composition of the present invention is not particularly limited, and a drug suitable for a target disease can be selected. Specific examples thereof include steroids such as betamethasone, dexamethasone, triamcinolone, prednisolone, fluorometholone, hydrocortisone and fluocinolone acetonide and derivatives thereof; hormones such as progesterone and testosterone and derivatives thereof; antiinflammatory agents such as bromofenac and diclofenac; cytokine inhibitors such as TNF-α inhibitors, anti-TNF-α antibodies, PDE-IV inhibitors and ICE inhibitors; immunosuppressive agents such as ciclosporin and tacrolimus; antiviral agents such as ganciclovir, acyclovir and interferon-β; antibacterial agents such as quinolone compounds, clarithromycin and erythromycin; ACE inhibitors such as captopril; HMG-CoA reductase inhibitors such as pravastatin and simvastatin; anticancer agents such as fluorouracil, methotrexate and MMP inhibitors; angiogenesis inhibitors such as endostatin, VEGF inhibitors, anti-VEGF antibodies, antisense oligonucleotides, PKC inhibitors, adhesion factor inhibitors and angiostatic steroids; neuroprotective agents and neurotrophic factors such as MK-801, timolol, creatine, taurine and BDNF; carbonate dehydratase inhibitors such as acetazolamide; thrombolytic agents such as urokinase; circulation improving agents; antifungal agents; antioxidants such as catechin and vitamin E; bone resorption inhibitors such as bisphosphonate; agents involved in the maintenance of visual function such as retinoic acid and derivatives thereof; and the like.

The present invention also relates to a method of improving transfer of a drug to a tissue of the posterior segment of the eye by a mucoadhesive substance, comprising administering a pharmaceutically effective amount of a solid composition containing the drug and the mucoadhesive substance in the conjunctival sac of a human patient, wherein the adhesion strength of the mucoadhesive substance is in the range of from 200 to 1000 g, and a method of treating an eye disease, comprising administering a therapeutically effective amount of a solid composition containing a drug and a mucoadhesive substance in the conjunctival sac of a human patient, wherein the adhesion strength of the mucoadhesive substance is in the range of from 200 to 1000 g, and transfer of the drug to a tissue of the posterior segment of the eye is improved by the mucoadhesive substance.

The amount of the drug to be incorporated in the solid composition of the present invention may be appropriately increased or decreased depending on the type of the drug, effective therapeutic concentration thereof, release period of the drug, symptoms or the like. The content of the drug is in the range of from 0.01 to 95% by weight, preferably from 0.1 to 30% by weight of the solid composition. The dose of the drug varies depending on the type of the drug, however, it is generally in the range of from about 1 µg to 100 mg per once.

The solid composition according to the present invention may be in the form capable of being administered in the conjunctival sac, and a special form with improved convenience upon administration or fitness after administration or the like is also included. Preferred examples of the form of the solid composition include a film form, a pellet form, a tablet form, a rod form, a particle form, a spherical form and the like. Such a composition can be prepared employing a commonly used technique. For example, it can be prepared using a method such as wet or dry compression molding, melt molding or solvent casting, and if necessary, any of various pharmaceutical additives such as an excipient, a binder, a disintegrant, a lubricant, a fluidizer, a stabilizer and a pH adjusting agent can be also added. Further, as a specific production example of the solid composition of the present invention, a solid composition using a carboxyvinyl polymer and hydroxypropyl cellulose as the mucoadhesive substances and the like will be shown in Examples mentioned below. The solid compositions prepared in Examples were evaluated by a method commonly used as a method of evaluating drug transfer to a tissue of the posterior segment of the eye simply and sensitively, that is, by using fluorescein or rhodamine B, which is a fluorescent dye, instead of a drug.

The solid composition to be used in the drug delivery system of the present invention may be administered in the conjunctival sac. Further, in order to further simplify the administration, an assistant device for exclusive use or the like can also be used. The administration of the solid composition does not require a professional technique such as intravitreal or subconjunctival injection or implant.

The administration frequency of the solid composition of the present invention can be appropriately selected depending on the symptoms, age, base or vehicle or the like, however, it may be administered once to several times (for example, once to six times) per day or once per several days to several months (one to several pieces of the solid composition at one time).

### Advantage of the Invention

As will be described in detail in the section of test below, by administering a solid composition containing a fluorescent dye and polyacrylic acid, a carboxyvinyl polymer, hydroxypropyl cellulose or a combination thereof as a mucoadhesive substance having an adhesion strength of 200 g or more in the conjunctival sac, an apparent difference in the concentration of the fluorescent dye present in the choroid and retina or vitreous body is observed between applied eyes and non-applied eyes. Further, it is confirmed that a solid composition containing a mucoadhesive substance shows apparently superior transfer of a fluorescent dye to a tissue of the posterior segment of the eye such as the choroid and retina or vitreous body to an eye drop, a solid composition not containing a mucoadhesive substance and a solid composition containing a mucoadhesive substance having an adhesion strength of less than 200 g. That is, by administering to a solid composition comprising a drug and a mucoadhesive substance having an adhesion strength of 200 g or more in the conjunctival sac, the direct drug transfer to a tissue of the posterior segment of the eye via a local eye tissue can be improved.

### Best Mode for Carrying Out the Invention

Hereinafter, preparation of a solid composition of the present invention, a test for measuring the concentration of a fluorescent dye in a tissue of the posterior segment of the eye, a test for measuring the adhesion strength of a mucoadhesive substance and preparation examples will be described, however, these examples are described for the purpose of understanding the present invention better and are not meant to limit the scope of the present invention.

### 1. Test 1

By using a solid composition (in the form of a pellet) containing a fluorescent dye and a mucoadhesive substance, and an eye drop containing the same type of fluorescent dye, transfer of the fluorescent dye to a tissue of the posterior segment of the eye via a local eye tissue was examined.

### 1-1. Production of preparation

### Example 1

Hydroxypropyl cellulose (hereinafter referred to as "HPC") and a carboxyvinyl polymer (hereinafter referred to as "CVP") were weighed such that the weight ratio thereof became 1:2, and ground and mixed homogeneously in a mortar. 95 mg of this powder was weighed, fluorescein (5 mg), which is a fluorescent dye, was added thereto, and these substances were further ground and mixed in a mortar. Subsequently, 75 mg of this powder was weighed and molded in the form of a disc by compression molding (10 kg/cm², 10 min). The thus prepared disc was cut into pieces with a size of 1 x 2 mm, whereby a HPC/CVP (1/2) preparation containing 5% (w/w) fluorescein (a solid composition in the form of a pellet) was obtained. Hereinafter, this preparation is referred to as Test preparation 1. Incidentally, as the HPC, hydroxypropyl cellulose manufactured by Wako Pure Chemical Industries, Ltd. was used, and as the CVP, carboxylvinyl polymer 934P manufactured by BASF was used.

### Comparative example 1

Fluorescein (16 mg), which is a fluorescent dye, was weighed into a mortar. After it was ground in the mortar, 2 mL of phosphate buffered saline containing 0.1% (w/w) polysorbate 80 was gradually added dropwise thereto to suspend it, whereby a suspension eye drop containing 0.8% (w/w) fluorescein was obtained. Hereinafter, this preparation is referred to as Comparative preparation 1.

### 1-2. Test method

Test preparation 1 prepared in Example 1 was administered in the conjunctival sac of one eye (right eye) of each rat (dose of fluorescein: 20 µg/eye). Then, rats were killed at 0.5, 1.5 and 4.5 hours after administration, both eyes, i.e., the applied eye and the non-applied eye were excised and the choroid and retina and the vitreous body were collected, respectively. The choroids and retinas collected from two eyes were combined to form one sample, and 250 µL of a mixed solution of methanol and water (1/1) was added thereto. After the mixture was homogenized and centrifuged (13000 rpm, 10 min), insoluble matter was removed. Then, the fluorescence intensity was measured (Em: 485 nm, Ex: 530 nm), and the fluorescein concentration in the choroid and retina was calculated. The vitreous bodies collected from two eyes were combined to form one sample, and a portion of 40 µL was taken out from the sample, and 200 µL of a mixed solution of methanol and water (1/1) was added thereto. After the mixture was homogenized and centrifuged (13000 rpm, 10 min), insoluble matter was removed. Then, the fluorescence intensity was measured (Em: 485 nm, Ex: 530 nm), and the fluorescein concentration in the vitreous body was calculated. The calculation of the fluorescein concentration was carried out using a calibration curve. The calibration curve was constructed by using a mixed solution of methanol and water (1/1) having fluorescein dissolved therein as a standard solution, adding the standard solution to the choroid and retina, and the vitreous body of normal eyes, and performing the same collection procedure as described above.

The blood was collected before the rats were killed in the above-mentioned procedure. The obtained sample was centrifuged (13000 rpm, 10 min), and insoluble matter was removed. Then, the fluorescence intensity was measured (Em: 485 nm, Ex: 530 nm), and the fluorescein concentration in the plasma was calculated. The calibration curve was constructed by using a mixed solution of methanol and water (1/1) having fluorescein dissolved therein as a standard solution, adding the standard solution to the plasma, and performing the same collection procedure as described above.

Further, Comparative preparation 1 was instilled into one eye (right eye) of each rat (dose of fluorescein: 40 µg/eye). Thereafter, the same procedure as described above was carried out, and the fluorescein concentration in the choroid and retina, the vitreous body and the plasma at 0.5, 1.5 and 4.5 hours after administration was calculated.

### 1-3. Test results and discussion

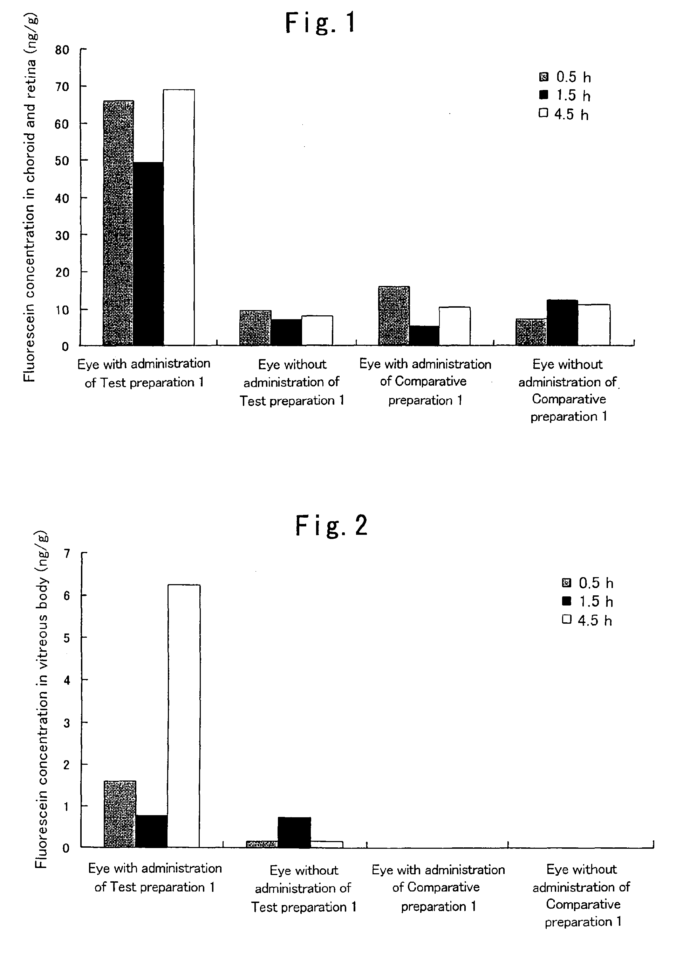

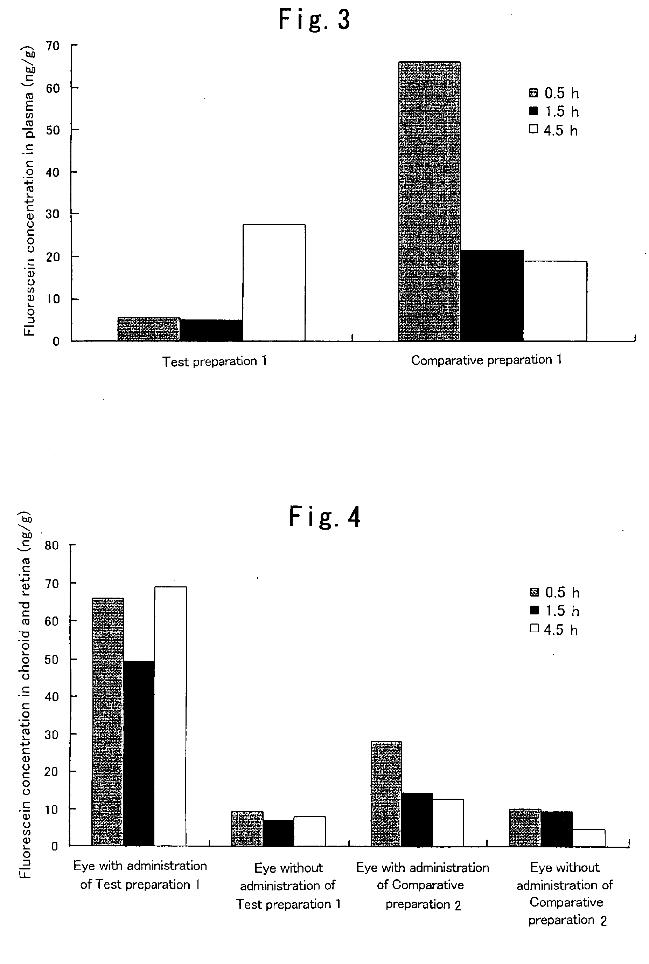

Fig. 1 shows the fluorescein concentration in the choroid and retina of the applied eye and the non-applied eye after a lapse of a predetermined time (0.5, 1.5 and 4.5 hours) after administration of Test preparation 1 or Comparative preparation 1 (case number: 6 eyes, 3 cases). Further, Fig. 2 and Fig. 3 show the fluorescein concentration in the vitreous body and the fluorescein concentration in the plasma after a lapse of a predetermined time (0.5, 1.5 and 4.5 hours) after administration of Test preparation 1 or Comparative preparation 1, respectively (case number: 6 eyes, 3 cases).

As is apparent from Fig. 1, while in Comparative preparation 1, a difference in the fluorescein concentration in the choroid and retina was almost not observed between the applied eye and the non-applied eye, in Test preparation 1, the fluorescein concentration in the choroid and retina of the applied eye showed an apparently higher value than in the non-applied eye. That is, it was found that by administering Test preparation 1, which is a solid composition comprising a mucoadhesive substance of the present invention, in the conjunctival sac, the fluorescent dye is efficiently transferred to a tissue of the choroid and retina.

As is apparent from Fig. 2, while in Comparative preparation 1, the fluorescein concentration in the vitreous body was not higher than the detection limit in both of the applied eye and the non-applied eye, in Test preparation 1, the fluorescein concentration in the vitreous body of the applied eye showed a higher value than in the non-applied eye. Particularly, an apparently high value was shown at 4.5 hours after administration. That is, it was found that by administering Test preparation 1, which is a solid composition containing a mucoadhesive substance of the present invention, in the conjunctival sac, the fluorescent dye is efficiently transferred to a tissue of the vitreous body.

As is apparent from Fig. 3, while in Comparative preparation 1, the fluorescein concentration in the plasma showed a high value from the early stage after administration, in Test preparation 1, the fluorescein concentration in the plasma showed a low value until at 1.5 hours after administration, and showed a high value only at 4.5 hours after administration. On the other hand, in Fig. 1 and Fig. 2, as described above, the fluorescein concentration in the choroid and retina and the vitreous body of the applied eye of Test preparation 1 showed an apparently higher value than in the non-applied eye, and a difference in the fluorescein concentration was not observed between the applied eye of Comparative preparation 1 and the non-applied eye. This indicates that the fluorescein contained in Test preparation 1, which is a solid composition comprising a mucoadhesive substance of the present invention, is transferred to a tissue of the posterior segment of the eye mainly via a local eye tissue, and Comparative preparation 1, which is a suspension eye drop, is transferred to a tissue of the posterior segment of the eye mainly via a systemic route. Incidentally, in Fig. 3, the reason why a high value was shown at 4.5 hours after administration in Test preparation 1 is considered to be because Test preparation 1 disintegrated at the administration site, resulting in an increase in fluorescein to be washed out with the lacrimal fluid.

From the above results, it was indicated that by administering a solid composition comprising a drug and a mucoadhesive substance in the conjunctival sac, the drug can be efficiently transferred to a tissue of the posterior segment of the eye such as a tissue of the choroid and retina or a tissue of the vitreous body, and the direct drug transfer to a tissue of the posterior segment of the eye via a local eye tissue is improved.

### 2. Test 2

By using a solid composition (in the form of a pellet) containing a mucoadhesive substance and a solid composition (in the form of a pellet) not containing a mucoadhesive substance, an effect of the presence or absence of the mucoadhesive substance on transfer of a fluorescent dye to a tissue of the posterior segment of the eye was examined. In Test 2, as the fluorescent dye to be incorporated in the solid composition, fluorescein was used in the same manner as in Test 1, and in Test 3 mentioned below, rhodamine B, which is another fluorescent dye, was used instead of fluorescein in order to reconfirm the importance of the mucoadhesive substance.

### 2-1. Production of preparation

### Comparative example 2

Fluorescein bulk powder (50 mg) and ethylene/vinyl acetate copolymer (hereinafter referred to as "EVA") (50 mg) were mixed by melting at 150 °C, the mixture was molded so as to have a thickness of about 1 mm, and the molded article was cut into pieces with a size of 1 x 2 mm, whereby an EVA preparation containing 50% (w/w) fluorescein (a solid composition in the form of a pellet) was obtained as a solid composition not containing a mucoadhesive substance. Hereinafter, this preparation is referred to as Comparative preparation 2. Incidentally, as the EVA, poly(ethylene-co-vinyl acetate), 33 wt% vinyl acetate manufactured by Aldrich Inc. was used.

### 2-2. Test method

By using Test preparation 1 and Comparative preparation 2, the same procedure as described in "1-2. Test method" was carried out, and the fluorescein concentration in the choroid and retina and the vitreous body at 0.5, 1.5 and 4.5 hours after administration was calculated. Test preparation 1 and Comparative preparation 2 were designed such that they showed a similar elution behavior.

### 2-3. Test results and discussion

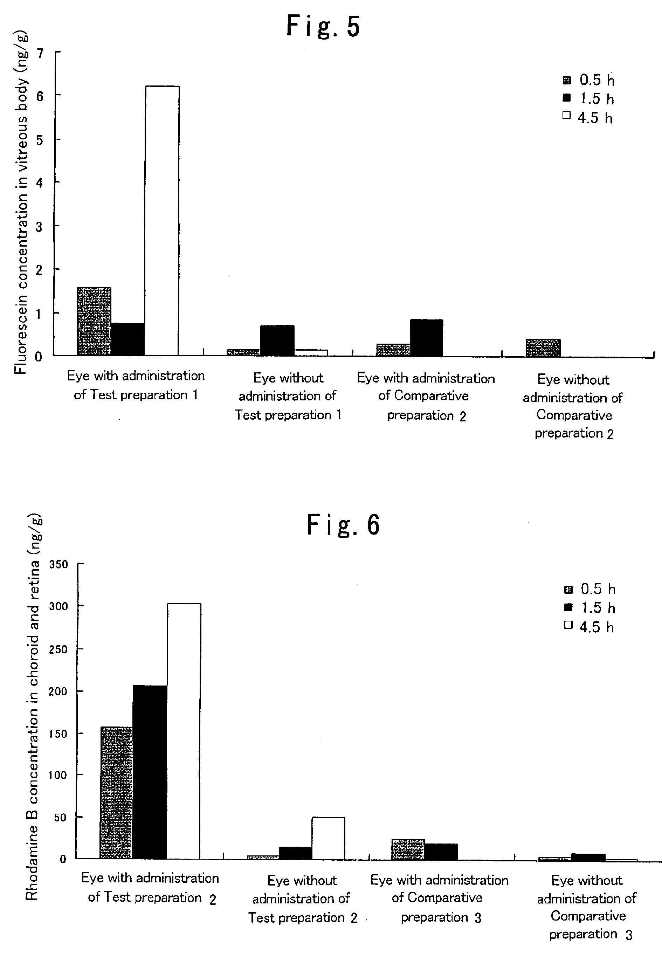

Fig. 4 shows the fluorescein concentration in the choroid and retina of the applied eye and the non-applied eye after a lapse of a predetermined time (0.5, 1.5 and 4.5 hours) after administration of Test preparation 1 or Comparative preparation 2 (case number: 6 eyes, 3 cases). Further, Fig. 5 shows the fluorescein concentration in the vitreous body of the applied eye and the non-applied eye after a lapse of a predetermined time (0.5, 1.5 and 4.5 hours) after administration of Test preparation 1 or Comparative preparation 2 (case number: 6 eyes, 3 cases).

As is apparent from Fig. 4, in Comparative preparation 2, although a small difference in the fluorescein concentration in the choroid and retina between the applied eye and the non-applied eye was observed at 0. 5 hours after administration, a difference was almost not observed at 1.5 and 4.5 hours after administration. On the other hand, in the applied eye of Test preparation 1, the fluorescein concentration in the choroid and retina showed an apparently higher value than in the non-applied eye. That is, it was found that a mucoadhesive substance to be incorporated in a solid composition is an important factor to promote the transfer of a fluorescent dye to a tissue of the choroid and retina via a local eye tissue.

As is apparent from Fig. 5, while in Comparative preparation 2, a difference in the fluorescein concentration in the vitreous body was almost not observed between the applied eye and the non-applied eye, in the applied eye of Test preparation 1, the fluorescein concentration in the vitreous body showed a higher value than in the non-applied eye. Particularly, an apparently high value was shown at 4.5 hours after administration. That is, it was found that a mucoadhesive substance to be incorporated in a solid composition is an important factor to promote the transfer of a fluorescent dye to a tissue of the vitreous body via a local eye tissue.

From the above results, it was indicated that a mucoadhesive substance to be incorporated in a solid composition is an important factor to efficiently transfer a drug to a tissue of the posterior segment of the eye such as a tissue of the choroid and retina or a tissue of the vitreous body, and to improve the direct drug transfer to a tissue of the posterior segment of the eye via a local eye tissue.

### 3. Test 3

By using rhodamine B, which is a fluorescent dye different from the dye used in Test 2, an effect of the presence or absence of a mucoadhesive substance on drug transfer to a tissue of the posterior segment of the eye was examined.

### 3-1. Production of preparation

### Example 2

HPC and CVP were weighed such that the weight ratio thereof became 1:2, and ground and mixed homogeneously in a mortar. 90 mg of this powder was weighed, rhodamine B (10 mg) was added thereto, and these substances were further ground and mixed in a mortar. Subsequently, 75 mg of this powder was weighed and molded in the form of a disc by compression molding (10 kg/cm², 10 min). The thus prepared disc was cut into pieces with a size of 1 x 2 mm, whereby a HPC/CVP (1/2) preparation containing 10% (w/w) rhodamine B (a solid composition in the form of a pellet) was obtained. Hereinafter, this preparation is referred to as Test preparation 2. Incidentally, as the HPC and CVP, the same compounds as in Example 1 were used.

### Comparative example 3

Rhodamine B (30 mg) and EVA (70 mg) were ground in a mortar and mixed by melting at 150 °C. Then, the mixture was molded so as to have a thickness of about 1 mm, and the molded article was cut into pieces with a size of 1 x 2 mm, whereby an EVA preparation containing 30% (w/w) rhodamine B (a solid composition in the form of a pellet) was obtained. Hereinafter, this preparation is referred to as Comparative preparation 3. Incidentally, as the EVA, the same compound as in Comparative example 2 was used.

### 3-2. Test method

By using Test preparation 2 and Comparative preparation 3, the same procedure as described in "1-2. Test method" was carried out, and the rhodamine B concentration in the choroid and retina and the vitreous body at 0.5, 1.5 and 4.5 hours after administration was calculated. In the test using Test preparation 2 and Comparative preparation 3, the fluorescence intensity was measured under the condition of Em: 530 nm and Ex: 580 nm. The calculation of the rhodamine B concentration was carried out using a calibration curve. The calibration curve was constructed by the same procedure as that for the calibration curve of fluorescein.

### 3-3. Test results and discussion

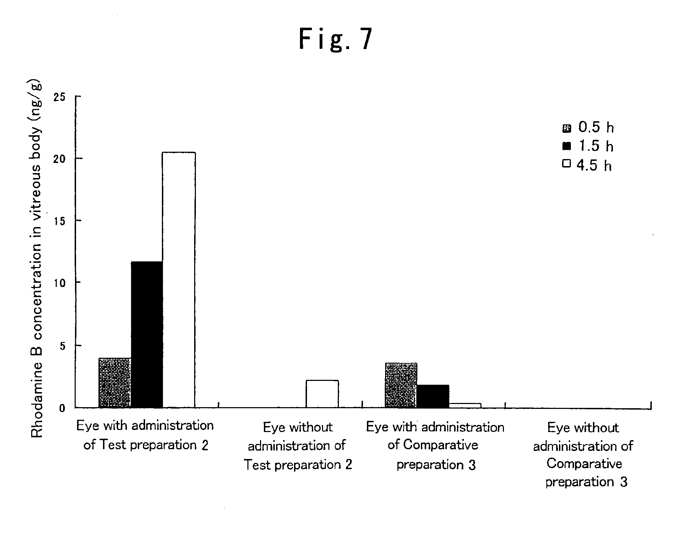

Fig. 6 shows the rhodamine B concentration in the choroid and retina of the applied eye and the non-applied eye after a lapse of a predetermined time (0.5, 1.5 and 4.5 hours) after administration of Test preparation 2 or Comparative preparation 3 (case number: 6 eyes, 3 cases). Further, Fig. 7 shows the rhodamine B concentration in the vitreous body of the applied eye and the non-applied eye after a lapse of a predetermined time (0.5, 1.5 and 4.5 hours) after administration of Test preparation 2 or Comparative preparation 3 (case number: 6 eyes, 3 cases).

As is apparent from Fig. 6, while in Comparative preparation 3, a difference in the rhodamine B concentration in the choroid and retina was almost not observed between the applied eye and the non-applied eye, in Test preparation 2, the rhodamine B concentration in the choroid and retina of the applied eye showed an apparently higher value than in the non-applied eye. Therefore, the same result as that in Test 2 was obtained that a solid composition containing a mucoadhesive substance shows superior transfer of a fluorescent dye to a tissue of the choroid and retina even when the fluorescent dye contained in the solid composition is different. That is, it was indicated that the mucoadhesive substance is an important factor to promote the transfer of a drug to a tissue of the choroid and retina via a local eye tissue. Further, because it was confirmed that also rhodamine B is efficiently transferred to a tissue of the choroid and retina via a local eye tissue, it was suggested that regardless of the compound to be incorporated in the solid composition, the mucoadhesive substance can promote the transfer thereof to a tissue of the choroid and retina via a local eye tissue.

As is apparent from Fig. 7, while in Comparative preparation 3, a difference in the rhodamine B concentration in the vitreous body was almost not observed between the applied eye and the non-applied eye, in Test preparation 2, the rhodamine B concentration in the vitreous body of the applied eye showed a higher value than in the non-applied eye. Therefore, the same result as that in Test 2 was obtained that a solid composition containing a mucoadhesive substance shows superior transfer of a fluorescent dye to a tissue of the vitreous body even when the fluorescent dye contained in the solid composition is different. That is, it was found that the mucoadhesive substance is an important factor to promote the transfer of a drug to a tissue of the vitreous body via a local eye tissue. Further, because it was confirmed that also rhodamine B is efficiently transferred to a tissue of the vitreous body via a local eye tissue, it was suggested that regardless of the compound to be incorporated in the solid composition, the transfer thereof to a tissue of the vitreous body via a local eye tissue can be promoted.

### 4. Test 4

By using solid compositions prepared using various mucoadhesive substances, transfer of a fluorescent dye to a tissue of the posterior segment of the eye was examined.

### 4-1. Production of preparation

### Example 3

HPC and CVP were weighed such that the weight ratio thereof became 2:1, and ground and mixed homogeneously in a mortar. 95 mg of this powder was weighed, fluorescein (5 mg), which is a fluorescent dye, was added thereto, and these substances were further ground and mixed in a mortar. Subsequently, 75 mg of this powder was weighed and molded in the form of a disc by compression molding (10 kg/cm², 10 min). The thus prepared disc was cut into pieces with a size of 1 x 2 mm, whereby a HPC/CVP (2/1) preparation containing 5% (w/w) fluorescein (a solid composition in the form of a pellet) was obtained. Hereinafter, this preparation is referred to as Test preparation 3. Incidentally, as the HPC and CVP, the same compounds as in Example 1 were used.

### Example 4

HPC (95 mg) and fluorescein (5 mg) were weighed into a mortar and were ground and mixed therein. Subsequently, 75 mg of this powder was weighed and molded in the form of a disc by compression molding (10 kg/cm², 10 min). The thus prepared disc was cut into pieces with a size of 1 x 2 mm, whereby a HPC preparation containing 5% (w/w) fluorescein (a solid composition in the form of a pellet) was obtained. Hereinafter, this preparation is referred to as Test preparation 4. Incidentally, as the HPC, the same compound as in Example 1 was used.

### Example 5

CVP (95 mg) and fluorescein (5 mg) were weighed into a mortar and were ground and mixed therein. Subsequently, 75 mg of this powder was weighed and molded in the form of a disc by compression molding (10 kg/cm², 10 min). The thus prepared disc was cut into pieces with a size of 1 x 2 mm, whereby a CVP preparation containing 5% (w/w) fluorescein (a solid composition in the form of a pellet) was obtained. Hereinafter, this preparation is referred to as Test preparation 5. Incidentally, as the CVP, the same compound as in Example 1 was used.

### Example 6

Polyacrylic acid (95 mg) and fluorescein (5 mg) were weighed into a mortar and were ground and mixed therein. Subsequently, 75 mg of this powder was weighed and molded in the form of a disc by compression molding (10 kg/cm², 10 min). The thus prepared disc was cut into pieces with a size of 1 x 2 mm, whereby a polyacrylic acid preparation containing 5% (w/w) fluorescein (a solid composition in the form of a pellet) was obtained. Hereinafter, this preparation is referred to as Test preparation 6. Incidentally, as the polyacrylic acid, polyacrylic acid AS-58 manufactured by Nippon Shokubai Co. Ltd. was used.

### Comparative example 4

Polylactic acid (hereinafter referred to as "PLA") and crystalline cellulose (hereinafter referred to as "MC") were weighed such that the weight ratio thereof became 1:1, and ground and mixed homogeneously in a mortar. 90 mg of this powder was weighed, fluorescein (5 mg) was added thereto, and these substances were further ground and mixed in a mortar. Subsequently, 75 mg of this powder was weighed and molded in the form of a disc by compression molding (10 kg/cm², 10 min). The thus prepared disc was cut into pieces with a size of 1 x 2 mm, whereby a PLA/MC (1/1) preparation containing 5% (w/w) fluorescein (a solid composition in the form of a pellet) was obtained. Hereinafter, this preparation is referred to as Comparative preparation 4. Incidentally, as the PLA, PLA 0005 manufactured by Wako Pure Chemical Industries, Ltd. was used, and as the MC, crystalline cellulose PH-101 manufactured by Asahi Kasei Corporation was used.

### Comparative example 5

Chitosan (95 mg) and fluorescein (5 mg) were weighed into a mortar and were ground and mixed therein. Subsequently, 75 mg of this powder was weighed and molded in the form of a disc by compression molding (10 kg/cm², 10 min). The thus prepared disc was cut into pieces with a size of 1 x 2 mm, whereby a chitosan preparation containing 5% (w/w) fluorescein (a solid composition in the form of a pellet) was obtained. Hereinafter, this preparation is referred to as Comparative preparation 5. Incidentally, as the chitosan, chitosan 500 manufactured by Wako Pure Chemical Industries, Ltd. was used.

### Comparative example 6

Sodium alginate (95 mg) and fluorescein (5 mg) were weighed into a mortar and were ground and mixed therein. Subsequently, 75 mg of this powder was weighed and molded in the form of a disc by compression molding (10 kg/cm², 10 min). The thus prepared disc was cut into pieces with a size of 1 x 2 mm, whereby a sodium alginate preparation containing 5% (w/w) fluorescein (a solid composition in the form of a pellet) was obtained. Hereinafter, this preparation is referred to as Comparative preparation 6. Incidentally, as the sodium alginate, Kimica Algin I-3 manufactured by Kimica Corporation was used.

### 4-2. Test method

By using Test preparations 3 to 6 and Comparative preparations 4 to 6, the same procedure as described in "1-2. Test method" was carried out, and the fluorescein concentration in the choroid and retina at 0.5, 1.5 and 4.5 hours after administration was calculated. Then, from the calculated data, AUC (area under the curve of the concentration in a tissue of the choroid and retina plotted against time) of the applied eye and the non-applied eye until 4.5 hours after administration of each preparation was obtained and its difference ((AUC of the applied eye) - (AUC of the non-applied eye), hereinafter referred to as "AUC_{4.5}") was calculated.

### 4-3. Test results and discussion

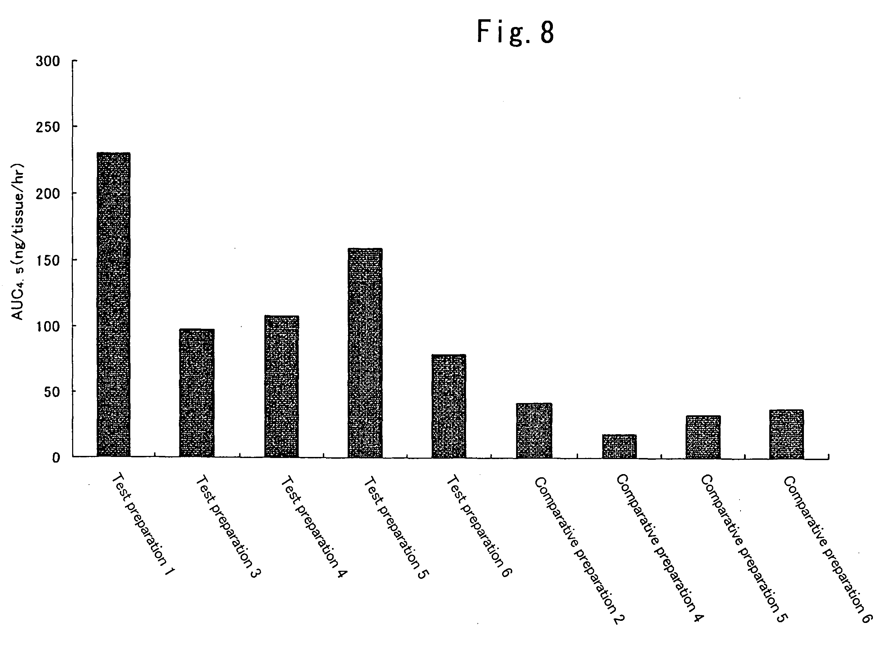

Fig. 8 shows the AUC_{4.5} of Test preparations 1, 3 to 6, and Comparative preparations 2 and 4 to 6 (case number: 2 to 6 eyes, 1 to 3 cases). Incidentally, as for the AUC_{4.5} of Test preparation 1 and Comparative preparation 2, AUC until 4.5 hours after administration of the applied eye of Test preparation 1 and Comparative preparation 2 and the non-applied eye was obtained by the same method as described in 4-2 based on the data obtained in Test 1 and Test 2, and its difference ((AUC of the applied eye) - (AUC of the non-applied eye)) was calculated (case number: 2 to 6 eyes, 1 to 3 cases).

As is apparent from Fig. 8, Test preparations 1 and 3 to 6, which are solid compositions containing a mucoadhesive substance, showed an apparently higher AUC_{4.5} value than Comparative preparation 2, which is a solid composition not containing a mucoadhesive substance. On the other hand, although Comparative preparations 4 to 6 are solid compositions containing a mucoadhesive substance, their AUC_{4.5} showed a value equal to or lower than that of Comparative preparation 2, which is a solid composition not containing a mucoadhesive substance. That is, it was indicated that among mucoadhesive substances, some substances show improvement of direct drug transfer to a tissue of the posterior segment of the eye via a local eye tissue, and some substances do not.

When comparison was performed with the data of adhesion strength of the mucoadhesive substances obtained in Test 5 mentioned below, although PLA/MC (1/1), chitosan and sodium alginate to be incorporated in Comparative preparations 4 to 6 were mucoadhesive substances, their adhesion strength was less than 200 g, however, the adhesion strength of HPC/CVP (1/2), HPC/CVP (2/1), HPC, CVP and polyacrylic acid to be incorporated in Test preparations 1 and 3 to 6 was 300 g or more. That is, it was indicated that for improving the direct drug transfer to a tissue of the posterior segment of the eye such as a tissue of the choroid and retina via a local eye tissue, the adhesion strength of the mucoadhesive substance to be incorporated in the solid composition is an important factor, and it was suggested that the mucoadhesive substance is required to have an adhesion strength of at least 200 g or more.

### 5. Test 5

The adhesion strength of various mucoadhesive substances was measured.

### 5-1. Preparation of compression-molded disc

Each mucoadhesive substance to be measured was ground and mixed in a mortar. In the case where particles with a large particle size were contained, they were removed by passing through a sieve with a mesh size of 500 µm. Subsequently, 75 mg of the resulting powder was weighed and placed in a mold and subjected to compression molding (10 kg/cm², 10 min), whereby a compression-molded disc was prepared. In the case where the mucoadhesive substance to be measured was a mixture, for example, in the case of HPC/CVP = 1/2, HPC and CVP were weighed such that the weight ratio became 1: 2, and were ground and mixed homogeneously in a mortar. Subsequently, 75 mg of the resulting mixed powder was weighed and placed in a mold (φ 13 mm) and subjected to compression molding (10 kg/cm², 10 min), whereby a compression-molded disc (φ 13 mm, thickness of 0.5 mm) was prepared.

By the above-mentioned procedure, compression-molded discs of HPC, CVP, HPC/CVP (1/2), HPC/CVP (2/1), polyvinyl alcohol (hereinafter referred to as "PVA"), hydroxypropyl methyl cellulose (hereinafter referred to as "HPMC"), sodium carboxymethyl cellulose (hereinafter referred to as "CMC-Na"), polyacrylic acid, PLA/MC (1/1), gellan gum, chitosan and sodium alginate were prepared. Incidentally, as the HPC, CVP, polyacrylic acid, PLA, MC, chitosan and sodium alginate, the same compounds as in Tests 1 to 4 were used. As the PVA, PVA 205 manufactured by Kuraray Co., Ltd. was used, as the HPMC, HPMC 2910 manufactured by Shin-Etsu Chemical Co., Ltd. was used, and as the CMC-Na, sodium carboxymethyl cellulose PR-S manufactured by Dai-ichi Kogyo Seiyaku Co., Ltd. was used.

### 5-2. Measurement method

Two silicone plugs (the surface with a smaller diameter: φ 14 mm) were prepared. One of the silicone plugs was placed such that the surface with a larger diameter was contacted with a bench (such as a laboratory bench) (i.e., the surface with a smaller diameter was faced upward), and 100 µL of physiological saline was placed dropwise on the upper surface (the surface with a smaller diameter) of the silicone plug. The compression-molded disc prepared in 5-1 was placed thereon, and 100 µL of physiological saline was further placed dropwise on the disc. Then, the other silicone plug was placed thereon such that the surface with a smaller diameter was contacted with the disc. After these members were allowed to stand still for 1 minute as such, they were compressed with a force of 667 g for a predetermined time (10 minutes) thereby to adhere to one another. By this procedure, a set of two silicone plugs having the compression-molded disc therebetween was prepared with two silicone plugs such that the surfaces with a smaller diameter were located to face each other. One of the two silicone plugs prepared in this way was fixed, and the other silicone plug was pulled with a force gauge (product name, manufactured by Imada Inc.). Then, the force when the adhesion of the two silicone plugs was released was measured. The value obtained by this procedure was determined to be the adhesion strength of a mucoadhesive substance. Incidentally, as the force gauge, force gauge DPS-0.5R (used when measuring an adhesion strength of 500 g or less, manufactured by Imada Inc.) and force gauge DPS-5 (used when measuring an adhesion strength of 500 g or more and 5 kg or less, manufactured by Imada Inc.) were used.

### 5-3. Measurement results

Table 1 shows the adhesion strengths (the value of adhesion strength is an average of 3 cases) of HPC, CVP, HPC/CVP (1/2), HPC/CVP (2/1), PVA, HPMC, CMC-Na, polyacrylic acid, PLA/MC (1/1), gellan gum, chitosan and sodium alginate, which are mucoadhesive substances.

### Table 1

**Table 1**

| Mucoadhesive substance | Adhesion strength (g) |
|---|---|
| HPC | 847 |
| CVP | 331 |
| HPC/CVP (1/2) | 362 |
| HPC/CVP (2/1) | 570 |
| PVA | 240 |
| HPMC | 311 |
| CMC-Na | 362 |
| Polyacrylic acid | 653 |
| PLA/MC (1/1) | 5 |
| Gellan gum | 5 |
| Chitosan | 5 |
| Sodium alginate | 194 |

### 6. Preparation examples

| Formulation example 1 (Pellet form) | (in 100 mg) |
|---|---|
| Triamcinolone | 10 mg |
| Hydroxypropyl cellulose | 30 mg |
| Carboxyvinyl polymer | 60 mg |

| Formulation example 2 (Pellet form) | (in 100 mg) |
|---|---|
| Triamcinolone | 10 mg |
| Polyacrylic acid | 90 mg |

### Brief Description of the Drawings

Fig. 1 is a graph showing the fluorescein concentration in the choroid and retina of the applied eye and the non-applied eye after a lapse of a predetermined time after administration of each of Test preparation 1 and Comparative preparation 1.
Fig. 2 is a graph showing the fluorescein concentration in the vitreous body after a lapse of a predetermined time after administration of each of Test preparation 1 and Comparative preparation 1.
Fig. 3 is a graph showing the fluorescein concentration in the plasma after a lapse of a predetermined time after administration of each of Test preparation 1 and Comparative preparation 1.
Fig. 4 is a graph showing the fluorescein concentration in the choroid and retina of the applied eye and the non-applied eye after a lapse of a predetermined time after administration of each of Test preparation 1 and Comparative preparation 2.
Fig. 5 is a graph showing the fluorescein concentration in the vitreous body of the applied eye and the non-applied eye after a lapse of a predetermined time after administration of each of Test preparation 1 and Comparative preparation 2.
Fig. 6 is a graph showing the rhodamine B concentration in the choroid and retina of the applied eye and the non-applied eye after a lapse of a predetermined time after administration of each of Test preparation 2 and Comparative preparation 3.
Fig. 7 is a graph showing the rhodamine B concentration in the vitreous body of the applied eye and the non-applied eye after a lapse of a predetermined time after administration of each of Test preparation 2 and Comparative preparation 3.
Fig. 8 is a graph showing a difference between the AUC (area under the curve of the concentration in a tissue of the choroid and retina plotted against time) of the applied eye and the AUC of the non-applied eye until 4.5 hours after administration of each of Test preparations 1, 3 to 6, and Comparative preparations 2 and 4 to 6.

## Claims

1. A noninvasive drug delivery system to a tissue of the posterior segment of the eye, **characterized by** administering a solid composition containing a drug and a mucoadhesive substance in the conjunctival sac, wherein the adhesion strength of the mucoadhesive substance is in the range of from 200 to 1000 g.

2. A solid composition, which contains a drug and a mucoadhesive substance and is to be administered in the conjunctival sac, wherein transfer of the drug to a tissue of the posterior segment of the eye is improved due to the incorporation of the mucoadhesive substance and the adhesion strength of the mucoadhesive substance of from 200 to 1000 g.

3. The drug delivery system according to claim 1 or the solid composition according to claim 2, wherein the adhesion strength of the mucoadhesive substance is in the range of from 300 to 900 g.

4. The noninvasive drug delivery system or solid composition according to claim 3, wherein the transfer of the drug to a tissue of the posterior segment of the eye is transfer via a local eye tissue.

5. The noninvasive drug delivery system or solid composition according to claim 3, wherein the transfer via a local eye tissue is transfer via a conjunctival tissue and a scleral tissue.

6. The noninvasive drug delivery system or solid composition according to claim 3, wherein the mucoadhesive substance is one member or a combination of a plurality of members selected from polyacrylic acid derivatives, cellulose derivatives and salts thereof.

7. The noninvasive drug delivery system or solid composition according to claim 6, wherein the polyacrylic acid derivative is a carboxyvinyl polymer or polyacrylic acid.

8. The noninvasive drug delivery system or solid composition according to claim 6, wherein the cellulose derivative is hydroxypropyl cellulose.

9. The noninvasive drug delivery system or solid composition according to claim 3, wherein the mucoadhesive substance is one member or a combination of a plurality of members selected from carboxyvinyl polymers and hydroxypropyl cellulose.

10. The noninvasive drug delivery system or solid composition according to claim 3, wherein the tissue of the posterior segment of the eye is a retina, a choroid, a sclera, an optic nerve, a tissue around the optic nerve or a vitreous body.

11. The noninvasive drug delivery system or solid composition according to claim 3, wherein the drug is a drug for treating or preventing a disease of a retina, a choroid, a sclera, an optic nerve, a tissue around the optic nerve or a vitreous body.

12. The noninvasive drug delivery system or solid composition according to claim 3, wherein the drug is an antiinflammatory agent, an immunosuppressive agent, an antiviral agent, an anticancer agent, an antithrombotic agent, an angiogenesis inhibitor, an optic neuroprotective agent, an antibacterial agent, an antifungal agent or an antioxidant.

13. The noninvasive drug delivery system or solid composition according to claim 3, wherein the solid composition is in the form of a film, a pellet, a tablet, a rod, a particle or a sphere.

14. A method of improving transfer of a drug to a tissue of the posterior segment of the eye by a mucoadhesive substance, comprising administering an amount of a solid composition containing the drug and the mucoadhesive substance in the conjunctival sac of a patient, wherein the adhesion strength of the mucoadhesive substance is in the range of from 200 to 1000 g.

15. A method of treating an eye disease, comprising administering a therapeutically effective amount of a solid composition containing a drug and a mucoadhesive substance in the conjunctival sac of a patient, wherein the adhesion strength of the mucoadhesive substance is in the range of from 200 to 1000 g, and transfer of the drug to a tissue of the posterior segment of the eye is improved by the mucoadhesive substance.

16. The method according to claim 14 or 15, wherein the adhesion strength of the mucoadhesive substance is in the range of from 300 to 900 g.

17. The method according to claim 16, wherein the transfer of the drug to a tissue of the posterior segment of the eye is transfer via a local eye tissue.

18. The method according to claim 17, wherein the transfer via a local eye tissue is transfer via a conjunctival tissue and a scleral tissue.

19. The method according to claim 16, wherein the mucoadhesive substance is one member or a combination of a plurality of members selected from polyacrylic acid derivatives, cellulose derivatives and salts thereof.

20. The method according to claim 19, wherein the polyacrylic acid derivative is a carboxyvinyl polymer or polyacrylic acid.

21. The method according to claim 19, wherein the cellulose derivative is hydroxypropyl cellulose.

22. The method according to claim 16, wherein the mucoadhesive substance is one member or a combination of a plurality of members selected from carboxyvinyl polymers and hydroxypropyl cellulose.

23. The method according to claim 16, wherein the tissue of the posterior segment of the eye is a retina, a choroid, a sclera, an optic nerve, a tissue around the optic nerve or a vitreous body.

24. The method according to claim 16, wherein the drug is a drug for treating or preventing a disease of a retina, a choroid, a sclera, an optic nerve, a tissue around the optic nerve or a vitreous body.

25. The method according to claim 16, wherein the drug is an antiinflammatory agent, an immunosuppressive agent, an antiviral agent, an anticancer agent, an antithrombotic agent, an angiogenesis inhibitor, an optic neuroprotective agent, an antibacterial agent, an antifungal agent or an antioxidant.

26. The method according to claim 16, wherein the solid composition is in the form of a film, a pellet, a tablet, a rod, a particle or a sphere.
